# EUROPÄISCHE PATENTSCHRIFT

(11) **EP 4 566 548 B1**
(45) Veröffentlichungstag und Bekanntmachung des Hinweises auf die Patenterteilung: **02.09.2026**
(21) Anmeldenummer: 24216997.7
(22) Anmeldetag: 03.12.2024
(51) Int. Cl.: A61B 17/00

(54) **BERGEBEUTEL UND LAPAROSKOPISCHE ANORDNUNG**
MINING BAGS AND LAPAROSCOPIC ASSEMBLY
SAC DE RECUPERATION ET ENSEMBLE LAPAROSCOPIQUE

(30) Priorität: 08.12.2023 DE 102023134402
(43) Veröffentlichungstag der Anmeldung: 11.06.2025
(73) Patentinhaber: BOWA-electronic GmbH & Co. KG, 72810 Gomaringen (DE)
(72) Erfinder: Kießling, Ingo, 72131 Ofterdingen (DE); Klein, Ralf, 72108 Rottenburg am Neckar (DE); Jinushi, Makoto, Kanagawa, 246-0025 (JP)
(74) Vertreter: Schneider, Peter Christian

(56) Entgegenhaltungen:
- EP-A2- 3 741 304
- KR-A- 20210 128 153

## Beschreibung

### Gebiet der Erfindung

Die Erfindung bezieht sich auf einen Bergebeutel mit einer flexiblen Beutelwand, die an ihrem oberen Ende von einer Beutelhauptöffnung begrenzt ist und mit ihrem unteren Drittel einen der Beutelhauptöffnung gegenüberliegenden Beutelboden bildet, an dem ein vom Beuteläußeren her zugängliches, flexibles Entroll-Zugmittel fixiert ist, welches länger ist als ein Abstand zwischen seinem Fixierungspunkt am Beutelboden und der Beutelhauptöffnung. Die relativen Positionsangaben "oben" und "unten" bzw. "oberes" und "unteres" beziehen sich auf die übliche bestimmungsgemäße Orientierung des funktionsbereiten Bergebeutels, bei dessen Befüllung Füllgut durch die Beutelhauptöffnung eingeführt werden und schwerkraftgetrieben von oben nach unten zum Beutelboden absinken kann.

Die Erfindung bezieht sich weiter auf eine laparoskopische Anordnung, umfassend
- einen Tubus und
- einen im Lumen des Tubus platzierten, derartigen Bergebeutel,
wobei der Bergebeutel parallel zur Beutelhauptöffnung zu einer Beutelrolle aufgerollt ist.

### Stand der Technik

Ein gattungsgemäßer Bergebeutel und eine gattungsgemäße laparoskopische Anordnung sind bekannt aus der EP 3 741 304 A2.

Bekanntermaßen stellt die Laparoskopie ein Teilgebiet der Chirurgie dar, bei dem Bauchoperationen ohne größeren Bauchschnitt durchgeführt werden. Stattdessen werden die notwendigen Operationsinstrumente über eine oder (meist) mehrere kleine Schnittlöcher in die Bauchhöhle eingeführt und die Operation wird unter Echtzeit-Videobeobachtung durchgeführt. Um die bei Exstirpationen anfallenden Gewebestücke zuverlässig und vollständig aus der Bauchhöhle entfernen zu können, kommen sogenannte Bergebeutel zum Einsatz. Es sind dies flexible Beutel, die in klein zusammengefaltetem oder aufgerolltem Zustand in die Bauchhöhle eingeführt und dort entfaltet werden, sodass die Gewebestücke beispielsweise mittels eines Greifinstruments durch die Beutelhauptöffnung in den Bergebeutel eingebracht werden können. Das Gewebestück kann dann sicher zusammen mit dem Bergebeutel durch eines der Schnittlöcher aus der Bauchhöhle entfernt werden. Hierbei sind unterschiedliche Detaillösungen bekannt, die beispielsweise ein (reversibles) Verschließen der Beutelhauptöffnung erlauben. Andere Detaillösungen sehen die Zerkleinerung des Gewebes innerhalb des Beutels mittels eines sogenannten Morcellators vor, wobei Varianten bekannt sind, bei denen der Bergebeutel eine zusätzlich zu seiner Beutelhauptöffnung vorgesehene Hilfsöffnung zur Einführung des Morcellators vorsieht.

Die WO 99/01068 A1 offenbart einen Bergebeutel, dessen Beutelhauptöffnung von einem Führungskanal aus umgeschlagener Beutelwand umgeben ist, wobei in besagtem Führungskanal ein federelastischer Draht verläuft. Dieser Draht wird in Vorbereitung einer laparoskopischen Operation zu einer sehr engen Ellipse verformt, sodass die Beutelhauptöffnung im Wesentlichen geschlossen ist. Sodann wird der Bergebeutel von seiner Beutelhauptöffnung her senkrecht zu und um besagte Drahtellipse herum aufgerollt, sodass er eine kleinvolumige, kompakte Beutelrolle bildet. Diese Beutelrolle wird in einen Tubus eingeschoben, in dessen Lumen ein kolbenartiger Schieber angeordnet ist, dessen Griff aus einem ersten Ende des Tubus herausragt. Ähnlich einer Kolbenspritze kann die Beutelrolle durch auf den Schieber ausgeübten Schub aus dem zweiten Ende des Tubus herausgeschoben werden. Ein derart mit Beutelrolle und Schieber versehener Tubus wird, wenn im Rahmen der laparoskopischen Operation der Einsatz eines Bergebeutels erforderlich wird, in ein Schnittloch in der Bauchdecke eingeführt, sodass sein erstes Ende mit dem Schieber für den Operateur zugänglich aus dem Schnittloch herausragt, während sein zweites, offenes Ende in die Bauchhöhle hineinragt. Durch Schubeinwirkung auf den Schieber relativ zum Tubus wird die Beutelrolle in die Bauchhöhle geschoben, wo sich die Beutelhauptöffnung aufgrund der Federkraft des Federdrahtes weitet. Dadurch soll sich zugleich der Bergebeutel entrollen.

Diese Doppelfunktion des primär als Offenhalter wirkenden Federdrahtes ist fehleranfällig. Insbesondere bei engen räumlichen Verhältnissen kann das Entrollen, bei dem sich das bei jedem Umlauf länger werdende, freie Ende des Beutels um die sich mit abnehmenden Druck der schwindenden Beutelrolle ausdehnende Federdraht-Schlaufe drehen muss, behindert werden. Verbleibt die Beutelrolle in einem unvollständigen Entrollzustand ist die Beutelhauptöffnung nicht zugänglich. Das Entwirren einer solchen Situation mittels mehrerer in die Bauchhöhle einzuführender Greifinstrumente ist heikel und zeitaufwendig.

Aus der eingangs genannten, gattungsbildenden EP 3 741 304 A2 ist es bekannt, zur Lösung dieses Problems den Beutel in umgekehrter Richtung, d.h. von seinem Beutelboden her aufzurollen und dabei einen am untersten Beutelrand fixierten Entrollfaden mit aufzurollen. Der Entrollfaden ist länger als der Beutel selbst, sodass sein freies Ende im Bereich der Beutelhauptöffnung aus der Beutelrolle herausragt.

Zug an dem Entroll-Zugmittel führt zwangsläufig zu einem Entrollen des Beutels von der Beutelhauptöffnung weg. Insbesondere bleibt diese in jedem Auf- bzw. Entrollzustand zugänglich, sodass selbst ein unvollständiges Entrollen den Zugang zur Beutelhauptöffnung nicht behindert. Außerdem wird die beim Entrollvorgang erforderliche mechanische Bewegung des Beutels auf ein Minimum reduziert. Insbesondere bedarf es keiner Rotation der Beutelhauptöffnung selbst. Diese kann vielmehr fixiert gehalten werden, z.B. mittels eines geeigneten Greifwerkzeugs oder, bei nach Art eines Keschers ausgebildetem Bergebeutel, mittels einer stielartigen Halterung. Die Beutelrolle, zu der dieser vorbekannte Bergebeutel im Kontext einer laparoskopischen Anordnung aufrollbar ist, ist mindestens ebenso kompakt wie Bergebeutel gleichen Volumens, die in umgekehrter Richtung, d.h. von der Beutelhauptöffnung zum Beutelboden hin, aufgerollt sind. In seinem unteren Mittenbereich weist der vorbekannte Bergebeutel eine Tülle auf, deren an der Tüllenspitze angeordnete, (noch) verschlossene Tüllenöffnung eine Hilfsöffnung des Bergebeutels darstellt, die beispielsweise zur Einführung eines Morcellators in den Bergebeutel genutzt werden kann. Um dabei den Austritt von Beutelinhalt in die Bauchhöhle zuverlässig zu vermeiden, ist es notwendig, dass besagte Tüllenöffnung im Rahmen der laparoskopischen Operation von außen unmittelbar zugänglich ist. Dies wird typischerweise dadurch bewerkstelligt, dass die Tüllenspitze mittels eines von außen durch eine Schnittöffnung in die Bauchhöhle eingeführten Greifinstrumentes gepackt und durch die Schnittöffnung nach außen gezogen wird, sodass die Tüllenöffnung für den Operateur unmittelbar zugänglich ist. Das ist aufwendig und erfordert erhebliches Geschick.

Die KR 2021 0128153 A offenbart ebenfalls einen aufrollbaren und mittels einer laparoskopischen Anordnung mit Tubus und Schieber applizierbaren Bergebeutel. Am Beutelboden ist ein biegsamer Draht befestigt, mittels dessen sich der gefüllte Bergebeutel umwickeln lässt, sodass das Bergegut im Beutel fixiert wird, um gemeinsam mit diesem entnommen werden zu können.

Die CN 2 18 589 047 U offenbart einen Bergebeutel mit einer im Bereich seines Beutelbodens angeordneten Tülle, durch welche ein Endoskop einführbar ist, mit dem Morcellationsprozesse im Beutelinneren beobachtet werden können.

Aus der DE 10 2016 114 786 A1 ist ein Saugkörper zur endoluminalen Unterdrucktherapie bekannt. Es handelt sich dabei um einen Schwammkörper, der das Ende eines im Umfassungsbereich porösen Schlauchs umfasst. Zur verbesserten Handhab- und Positionierbarkeit sind der Schwammkörper oder das Schlauchende mit einer Fassschlaufe ausgestattet.

### Aufgabenstellung

Es ist die Aufgabe der vorliegenden Erfindung, einen verbesserten Bergebeutel und eine verbesserte laparoskopische Anordnung zur Verfügung zu stellen, welche eine einfachere Nutzung einer Tülle des Bergebeutels zulassen.

### Darlegung der Erfindung

Diese Aufgabe wird in Verbindung mit den Merkmalen des Oberbegriffs von Anspruch 1 dadurch gelöst, dass der Beutelboden die Tülle aufweist und das Entroll-Zugmittel an der Tülle fixiert ist.

Die Aufgabe wird weiter durch eine laparoskopische Anordnung mit den Merkmalen von Anspruch 9 gelöst, d. h. durch eine laparoskopische Anordnung, umfassend
- einen Tubus und
- einen im Lumen des Tubus platzierten, erfindungsgemäßen Bergebeutel,
wobei der Bergebeutel senkrecht zur Beutelhauptöffnung zu einer Beutelrolle derart aufgerollt ist, dass das Entroll-Zugmittel in der vom Beutelboden her aufgerollten Beutelrolle parallel zur Beutelwand verlaufend mit aufgerollt ist, sodass sein freies Ende im Bereich der Beutelhauptöffnung aus der Beutelrolle herausragt.

Bevorzugte Ausführungsformen sind Gegenstand der abhängigen Ansprüche.

Die Erfindung übernimmt den aus dem Stand der Technik bekannten Grundgedanken, am Beutelboden, der hier als der gesamte Bereich des unteren, d.h. der Beutelhauptöffnung gegenüberliegenden Drittels der Beutelwand definiert ist, ein vom Beuteläußeren her zugängliches Entroll-Zugmittel, beispielsweise einen Faden oder ein Band, anzubringen, welches sich bei der Herstellung einer kompakten Beutelrolle gemeinsam mit dem Beutel, insbesondere an der Außenseite seiner Beutelwand anliegend, aufrollen lässt und aufgrund seiner vorgegebenen Mindestlänge mit seinem freien Ende aus besagter Beutelrolle hervorragt. Insbesondere kann der Beutel vom Beutelboden her in Richtung auf die Beutelhauptöffnung aufgerollt sein bzw. werden. Damit wird der eingangs bereits beschriebene Entrollmechanismus verwirklicht

Zwar steht für die Anbringung des Entroll-Zugmittels der gesamte untere Bereich des Bergebeutels, d.h. der gesamte Beutelboden, und für die Anbringung der Tülle die gesamte Beutelwand zur Verfügung. Erfindungsgemäß ist es jedoch vorgesehen, dass der Beutelboden die Tülle aufweist, wobei das Entroll-Zugmittel an besagter Tülle fixiert ist. Die ggf. bereits vorhandene oder in die anfänglich geschlossene Tüllenspitze einbringbare Tüllenöffnung stellt die eingangs bereits beschriebene Hilfsöffnung zusätzlich zur Beutelhauptöffnung dar, wobei sich die erfindungsgemäße Fixierung des Entroll-Zugmittels an besagter Tülle als besonders vorteilhaft erweist: Wird nämlich der Entroll-Zug mittels eines Werkzeugs auf das Entroll-Zugmittel aufgebracht, welches durch einen Bauchschnitt in die Bauchhöhle eingeführt wird, kann das Entroll-Zugmittel und sodann auch die mit ihm verbundene Tülle durch eben jenen Bauchschnitt nach außen gezogen werden, sodass die Tüllenöffnung zur bequemen Einführung des Morcellators zur Verfügung steht. Der in der Bauchhöhle verbleibende Hauptteil des Bergebeutels bleibt dabei weitgehend unverformt. Es genügt, wenn die Tülle eine flexible Ausstülpung der Beutelwand bildet. Eine steife Gestaltung der Tülle ist möglich aber nicht zwingend erforderlich.

Bevorzugt ist, wie aus dem Stand der Technik grundsätzlich bekannt, im Lumen des Tubus der Bergerolle benachbart ein Schieber angeordnet, der aus einem ersten Ende des Tubus herausragt, sodass die Beutelrolle durch bei festgehaltenem Tubus auf den Schieber ausgeübten Schub am zweiten Ende des Tubus aus diesem herausschiebbar ist. Der Schieber kann unabhängig von der Beutelrolle kolbenartig ausgebildet sein. Bei einer solchen Ausführungsform lässt sich die Beutelrolle aus dem Tubus auswerfen und im weiteren Verlauf unabhängig von diesem und unabhängig vom Schieber handhaben. Der Tubus steht dann als Zugang zur Einführung anderer Instrumente, beispielsweise eines Greifwerkzeugs, eines Schneidwerkzeugs oder einer Optik zur Verfügung. Es ist sogar möglich, ein solches Greif- oder Schneidwerkzeug selbst zunächst als Schieber und nach dem Auswerfen der Beutelrolle in seiner eigentlichen Funktion zu verwenden.

Bei einer alternativen Ausführungsform ist vorgesehen, dass der Schieber im Bereich der Beutelhauptöffnung an der Beutelwand fixiert ist. Diese Fixierung kann mittelbar oder unmittelbar erfolgen. Die Idee hinter dieser hier bildlich als "kescherartig" beschriebenen Gestaltung ist es, dem Schieber eine Doppelfunktion zuzuweisen: nämlich einerseits als eigentlicher Schieber, mittels dessen die Beutelrolle aus dem Tubus herausgeschoben wird, und andererseits als Halter für den Bergebeutel. Insbesondere kann das Entrollen des Bergebeutels durch Zug an dem Entroll-Zugmittel dadurch erleichtert werden, dass die aufgebrachte Zugkraft über den Halter/Schieber abgestützt wird. Ein separates Greifwerkzeug zur Aufbringung dieser Abstützkraft ist bei dieser Gestaltung nicht erforderlich.

In jedem Fall jedoch kann eine erfindungsgemäße laparoskopische Anordnung wie folgt benutzt werden, nämlich im Rahmen eines Verfahrens, umfassend die Schritte:
a) Einführen des Tubus in eine Wandungsöffnung einer einen Hohlraum umgebenden Wand, sodass das erste Ende des Tubus nach außen aus der Wandungsöffnung herausragt und das zweite Ende des Tubus in den Hohlraum hineinragt,
b) Ausüben eines axialen Schubs auf die Beutelrolle, bevorzugt mittels eines aus dem ersten Ende des Tubus herausragenden Schiebers, sodass die Beutelrolle am zweiten Ende des Tubus aus diesem heraus in den Hohlraum geschoben wird,
c) Erfassen des freien Endes des Zugmittels mittels eines ersten Greifwerkzeugs und
d) Ausüben eines Zugs an dem freien Ende des Zugmittels.

Wie oben bereits erwähnt, kann der (funktionale) Schieber durch ein speziell hierfür vorgesehenes und bevorzugt unverlierbar mit dem Tubus verbundenes Element oder aber durch ein im Folgenden dann als solches verwendetes Greif-, Schneid- oder anderes, Werkzeug, insbesondere ein chirurgisches Instrument, realisiert sein.

Wie ebenfalls bereits erwähnt, kann das Entroll-Zugmittel vorzugsweise als Faden oder Band ausgestaltet sein. Insbesondere wird es als vorteilhaft angesehen, wenn das Entroll-Zugmittel als mehrsträngiger Faden oder mehrsträngiges Band ausgebildet ist, dessen freie Strangenden zur Bildung wenigstens einer Schlaufe miteinander verbunden sind oder ineinander übergehen. Durch eine solche Gestaltung wird es ermöglicht, die zum Entrollen erforderliche Zugkraft mittels eines Hakens anstelle eines zangenartigen Greifwerkzeugs auf das Entroll-Zugmittel aufzubringen. Die fixierten Enden des mehrsträngigen Fadens oder Bandes können bereits in einigem Abstand von ihrer Fixierungsstelle am Beutelboden ineinander über gehen, sodass sich eine lassoartige Gestaltung ergibt. Denkbar ist freilich auch die Anbringung eines Ringes oder einer Öse am freien Ende des Entroll-Zugmittels.

Günstigerweise trägt die Beutelhauptöffnung eine umlaufende Wandverstärkung. Dies ist günstig um eine gute Definition des geöffneten Zustandes des Beutels zu erzielen und um ein wohldefiniertes Widerlager zur Abstützung der Entroll-Zugkraft zur Verfügung zu stellen.

Besonders bevorzugt ist die Wandverstärkung als ein federelastischer Offenhalter ausgebildet, der in einem die Beutelhauptöffnung umlaufenden Führungskanal geführt und so vorgeformt ist, dass er im kraftunbeaufschlagten Zustand einen Ring bildet. Beispielsweise kann der Offenhalter als ein Federdrahtring ausgebildet sein, wie dies im Stand der Technik grundsätzlich bekannt ist. Ein solcher Federdrahtring lässt sich zu einer sehr schmalen Ellipse verformen, die als Teil der Beutelrolle in den Tubus der erfindungsgemäßen laparoskopischen Anordnung einschiebbar ist und sich nach dem Herausschieben der Beutelrolle selbsttätig entfaltet und eine leichte Zugänglichkeit des Beutelinneren durch die Beutelhauptöffnung zulässt.

Der von dem Offenhalter im kraftunbeaufschlagten Zustand gebildete Ring ist bevorzugt als ein offener Ring mit einem relativ zur Beutelwand festen, ersten Ende und einem freien, zweiten Ende ausgebildet, sodass die lichte Weite der Beutelhauptöffnung durch bei festgehaltenem ersten Ende auf das zweite Ende einwirkenden Zug verringerbar ist. Mit anderen Worten bildet der Offenhalter eine federelastische Schlinge, mittels welcher die Beutelhauptöffnung zugezogen werden kann und sich günstigerweise bei Wegfall einer Zugkraft wieder selbsttätig weitet. Ein oder beide proximalen Enden des offenen Rings können bei hinreichender Steifigkeit zugleich als Schieber der erfindungsgemäßen laparoskopischen Anordnung ausgebildet sein, womit diesem Element drei Funktionen, nämlich als eigentlicher Schieber, als Halter für den Bergebeutel und als Betätigungselement für das Öffnen und Verschließen der Beutelhauptöffnung zugewiesen werden.

Weitere Einzelheiten und Vorteile der Erfindung ergeben sich aus der nachfolgenden speziellen Beschreibung und den Zeichnungen.

### Kurzbeschreibung der Zeichnungen

Es zeigen:
- Figur 1:: eine schematische Darstellung eines erfindungsgemäßen Bergebeutels in verschiedenen Ausführungsformen,
- Figur 2:: der Bergebeutel von Figur 1 in Vorbereitung der Herstellung einer Beutelrolle,
- Figur 3:: eine Beutelrolle in Vorbereitung der Herstellung einer erfindungsgemäßen laparoskopischen Anordnung,
- Figur 4:: eine erfindungsgemäße laparoskopische Anordnung,
- Figur 5:: eine erste Alternative zur Beutelrolle von Figur 3,
- Figur 6:: eine zweite Alternative zur Beutelrolle von Figur 3 sowie
- Figur 7:: eine dritte Alternative zur Beutelrolle von Figur 3.

### Beschreibung bevorzugter Ausführungsformen

Gleiche Bezugszeichen in den Figuren deuten auf gleiche oder analoge Elemente hin.

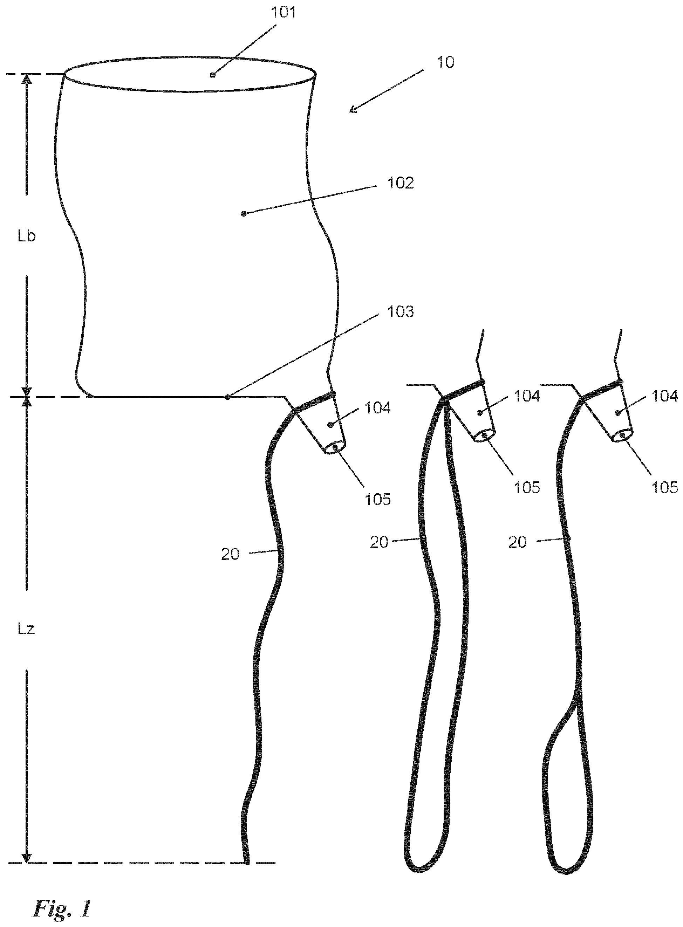

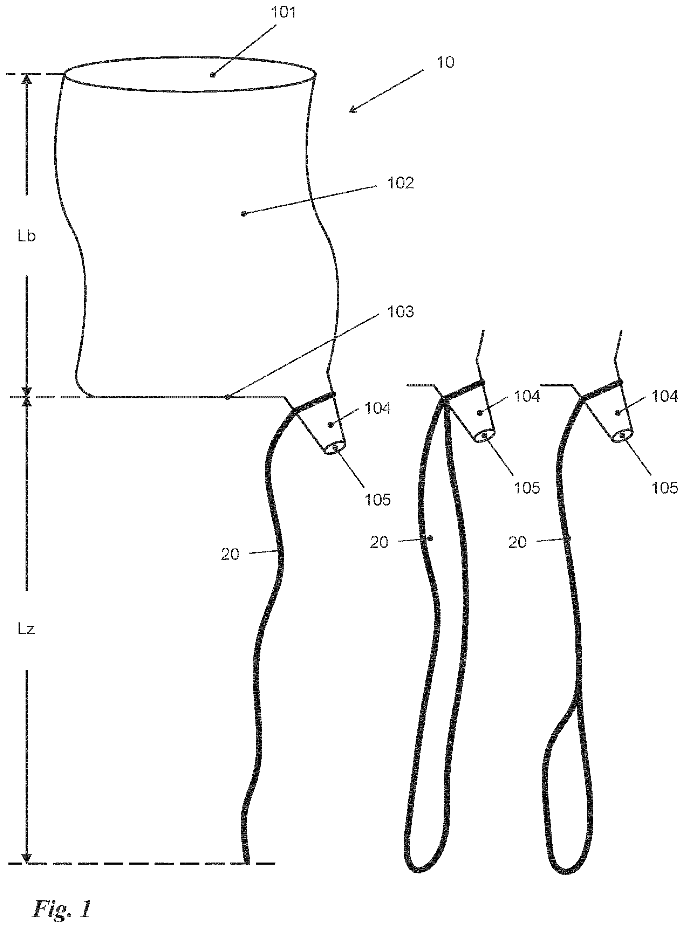

Figur 1 zeigt in stark schematisierter Darstellung einen erfindungsgemäßen Bergebeutel 10. Der Bergebeutel 10 weist eine Beutelhauptöffnung 101 auf, die einen offenen Abschluss einer sie vollumfänglich umlaufenden Beutelwand 102 bildet. Die Beutelwand 102 erstreckt sich senkrecht zur Beutelhauptöffnung 101 bis zu einem dieser gegenüberliegenden, im Wesentlichen geschlossenen Beutelende, wo sie einen Beutelboden 103 bildet. Bei der dargestellten Ausführungsform weist der Beutelboden 103 eine hier als Tülle 104 bezeichnete, flexible Ausstülpung der Beutelwand 102 auf, an deren Ende sich eine Tüllenöffnung 105 befindet, sodass die Tülle 104 einen zu der Beutelhauptöffnung 101 zusätzlichen, kanalartigen Zugang zum Inneren des Bergebeutels 10 bildet. Bei alternativen Ausführungsformen ist die Tülle 104 anfänglich verschlossen und wird erst bei Bedarf, z.B. durch Abschneiden der Tüllenspitze, geöffnet.

An der Tülle 104 ist ein als Faden oder Band ausgebildetes Entroll-Zugmittel 20 fixiert. Wie in Figur 1 dargestellt, ist die Länge Lz des Entroll-Zugmittels 20 größer als die Länge Lb des Bergebeutels 10, d.h. länger als der Abstand zwischen der Beutelhauptöffnung 110 und seinem Fixierungspunkt am Beutelboden 103 bei entrolltem Bergebeutel 10, wie in Figur 1 dargestellt. Zur Verringerung dieses Abstandes und damit zur Verringerung der erforderlichen Zugmittel-Länge kann die Tülle 104 um ihre Ansatzlinie an der Beutelwand 103 umgeklappt oder umgefaltet werden. Dies bietet sich insbesondere bei Ausführungsformen an, bei denen die Tülle nicht als eigenstabilier Körper, sondern aus zwei randständig miteinander verbundenen Folien hergestellt ist.

Der Bereich der Tülle 104 und des Entroll-Zugmittels 20 ist in Figur 1 mehrfach dargestellt, um einige von verschiedenen möglichen Gestaltungsabwandlungen des Entroll-Zugmittels 20 zu illustrieren. Insbesondere ist in den zusätzlichen Darstellungen das Entroll-Zugmittel schlaufenartig ausgebildet, wobei die die Schlaufe bildenden Teilstränge unmittelbar an der Tülle 4 oder bereits beabstandet von dieser zusammenlaufen können. Eine weitere Alternative besteht darin, das freie Ende des Entroll-Zugmittels 20 mit einem Ring oder einer Öse zu versehen.

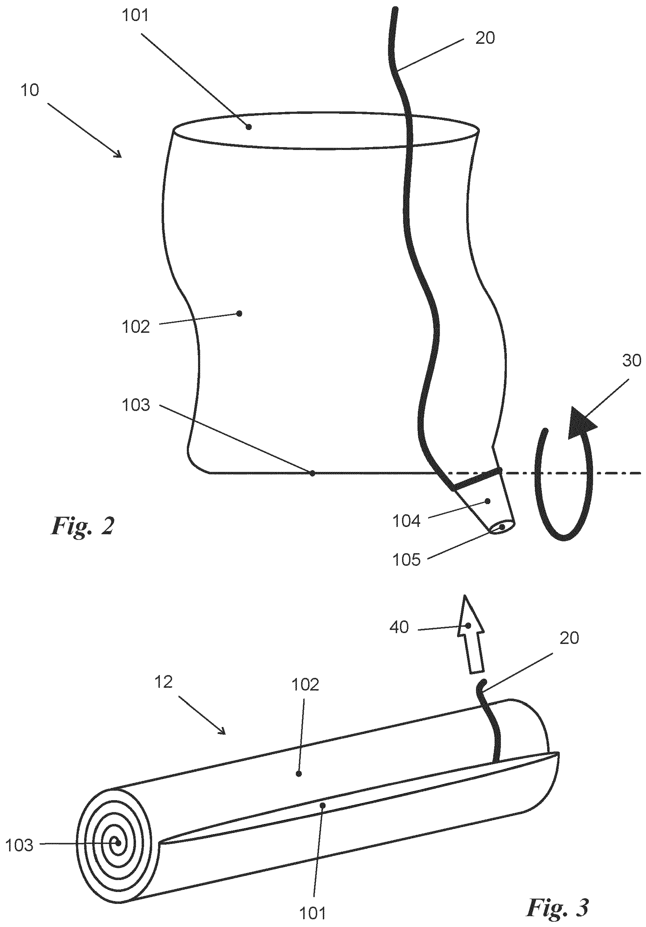

Figur 2 zeigt den Bergebeutel von Figur 1 in einem Vorbereitungszustand für den Aufbau einer erfindungsgemäßen laparoskopischen Anordnung, insbesondere deren Beutelrolle. Hierzu wird das Entroll-Zugmittel 20 von seinem Ansatzpunkt an der Tülle 104 parallel zur Beutelwand 102 bis zur Beutelhauptöffnung 101 und über diese hinaus verlegt. Sodann wird der Bergebeutel, wie durch den Rotationspfeil 30 angedeutet, um seine beutelbodenseitige Kante herum aufgewickelt, wobei das parallel zur Beutelwand 102 verlegte Entroll-Zugmittel 20 mit aufgerollt wird.

Das Ergebnis ist die in Figur 3 schematisch dargestellte Beutelrolle 12. Man erkennt das im Bereich der Beutelhauptöffnung 101 herausragende, freie Ende des Entroll-Zugmittels 20. Durch Zug an diesem freien Ende, wie durch den Zugpfeil 40 angedeutet, lässt sich die Beutelrolle 12 vollständig aufrollen und in ihren in Figur 1 dargestellten, entrollten Zustand überführen.

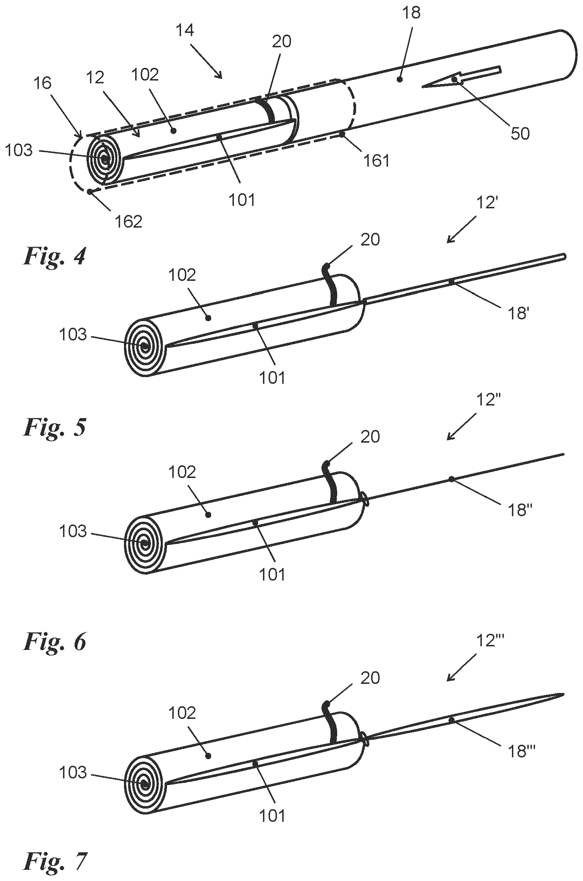

Die Beutelrolle 12 ist ein wesentlicher Bestandteil der in Figur 4 grob schematisiert dargestellten, erfindungsgemäßen laparoskopischen Anordnung 14. Diese umfasst einen der besseren Erkennbarkeit halber in Figur 4 nur gestrichelt dargestellten Tubus 16, in den die Beutelrolle 12 vorzugsweise in ihrer gesamten Länge eingesetzt ist. Die durch den Tubus 16 vorgegebene Volumenbeschränkung verhindert ein selbsttätiges Entrollen der Beutelrolle 12.

Weiter umfasst die laparoskopische Anordnung 14 einen Schieber 18, der nur teilweise in den Tubus 16 eingeschoben ist. Es kann sich hierbei um ein spezialisiertes und vorzugsweise verliersicher mit dem Tubus 16 verbundenes Element oder aber um ein Instrument mit anderem Primärnutzen, z.B. um eine chirurgisches Greif- oder Schneidinstrument handeln, das lediglich temporär als Schieber 18 zweckentfremdet wird. Im Rahmen der vorliegenden Beschreibung wird dasjenige Ende des Tubus 16, in welchen der Schieber 18 eingeschoben ist, als erstes Ende 161 und das gegenüberliegende Tubusende als zweites Ende 262 des Tubus 16 bezeichnet. Bei einem gemäß dem Schubpfeil 50 auf den Schieber 18 angewandten Schub kann die Beutelrolle 12 aus dem zweiten Ende 162 des Tubus 16 herausgeschoben werden. Die bevorzugte Anwendung eines derartigen Mechanismus im Rahmen einer laparoskopischen Operation ist im allgemeinen Teil der Beschreibung ausführlich erläutert.

Die Figuren 5 bis 7 zeigen unterschiedliche Abwandlungen der Beutelrolle 12 von Figur 3. Im Fall der Beutelrolle 12' von Figur 5 ist ein stabartiger Schieber 18' an der Beutelrolle 12', insbesondere an der Beutelhauptöffnung 101 fixiert. Der Schieber 18' erfüllt damit eine Doppelfunktion, nämlich zum einen als eigentlicher Schieber und zum anderen als Halter für die Beutelrolle 12' (bzw. nach deren Entrollen für den Bergebeutel 12). Insbesondere für den Schritt des Entrollens durch Zug am freien Ende des Entroll-Zugmittels 20 erlaubt es der integrierte Schieber 18', eine der Zugkraft entgegenwirkende Abstützkraft im Bereich der Beutelhauptöffnung 101 aufzubringen. Dies kann zusätzlich dadurch unterstützt werden, dass die Beutelhauptöffnung 101 mit einer in den Figuren nicht gesondert dargestellten, versteifenden Wandverstärkung versehen ist.

Im Fall der Beutelrolle 12" von Figur 6 ist der Schieber 18" ebenfalls im Bereich der Beutelhauptöffnung fixiert. Insbesondere wird er bei dieser Ausführungsform durch einen zugsteifen, biegeelastischen Federdraht realisiert, der die gesamte Beutelhauptöffnung 101 in einem nicht gesondert dargestellten Führungskanal umläuft, um einen offenen Ring zu bilden, der allerdings im aufgerollten Zustand der Beutelrolle 12", wie in Figur 6 gezeigt, zu einer schmalen Ellipse zusammengedrückt ist. Der Schieber 18" erfüllt hier drei Funktionen, nämlich die eigentliche Schieberfunktion, die im Kontext von Figur 5 bereits beschriebene Halterfunktion und die Funktion eines Verschluss-Zugmittels. Nach dem Herausschieben der Beutelrolle 12" aus dem Tubus 16 einer entsprechenden laparoskopischen Anordnung 14 und insbesondere während oder nach dem Entrollen wird sich die Beutelhauptöffnung 101 aufgrund der elastischen Federkraft des Federdrahtes selbsttätig öffnen. Wird jedoch auf das freie Ende des Schiebers 18" ein Zug aufgebracht, während zugleich sein anderes, am Bergebeutel 10 fixiertes Ende festgehalten wird, lässt sich die Beutelhauptöffnung 101 schlingenartig zuziehen.

Eine ähnliche Funktionsweise liegt bei der Beutelrolle 12‴ von Figur 7 vor. Hier ist der integrierte, tripelfunktionale Schieber 18‴ allerdings als ein zu einer "8" verdrillter Federdrahtring ausgestaltet, der durch eine Öse 181 im Bereich der Beutelhauptöffnung 101 geführt ist. Der in Figur 7 rechts dargestellte, freie 8-Bauch dient als Griff des Schiebers 18"'; der zweite 8-Bauch umläuft die Beutelhauptöffnung in einem Führungskanal, wie bereits im Kontext von Figur 6 beschrieben. Zug an dem Griff des Schiebers 18‴ führt bei festgehaltener Öse 181 zu einer Verengung der Beutelhauptöffnung 101. Im Übrigen kann auf die Ausführungen zu Figur 6 verwiesen werden.

Natürlich stellen die in der speziellen Beschreibung diskutierten und in den Figuren gezeigten Ausführungsformen nur illustrative Ausführungsbeispiele der vorliegenden Erfindung dar.

### Bezugszeichenliste

- 10: Bergebeutel
- 101: Beutelhauptöffnung
- 102: Beutelwand
- 103: Beutelboden
- 104: Tülle
- 105: Tüllenöffnung
- 12: Beutelrolle
- 12': Beutelrolle
- 12": Beutelrolle
- 12‴: Beutelrolle
- 14: laparoskopische Anordnung
- 16: Tubus
- 161: erstes Ende von 16
- 162: zweites Ende von 16
- 18: Schieber
- 18': Schieber
- 18": Schieber
- 18‴: Schieber
- 20: Entroll-Zugmittel
- 30: Rotationspfeil
- 40: Zugpfeil

## Patentansprüche

1. Bergebeutel (10) mit einer flexiblen Beutelwand (102), die an ihrem oberen Ende von einer Beutelhauptöffnung (101) begrenzt ist, wenigstens eine Tülle (104) aufweist und mit ihrem unteren Drittel einen der Beutelhauptöffnung (101) gegenüberliegenden Beutelboden (103) bildet, an dem
ein vom Beuteläußeren her zugängliches, flexibles Entroll-Zugmittel (20) fixiert ist, welches länger ist als ein Abstand zwischen seinem Fixierungspunkt am Beutelboden (103) und der Beutelhauptöffnung (101),
**dadurch gekennzeichnet,**
**dass** der Beutelboden (103) die Tülle (104) aufweist und das Entroll-Zugmittel (20) an der Tülle (104) fixiert ist.

2. Bergebeutel (10) nach Anspruch 1,
**dadurch gekennzeichnet,**
**dass** das Entroll-Zugmittel (20) als Faden oder Band ausgebildet ist.

3. Bergebeutel (10) nach Anspruch 2,
**dadurch gekennzeichnet,**
**dass** das Entroll-Zugmittel (20) als mehrsträngiger Faden oder mehrsträngiges Band ausgebildet ist, dessen freie Strangenden zur Bildung wenigstens einer Schlaufe miteinander verbunden sind oder ineinander übergehen.

4. Bergebeutel (10) nach einem der vorangehenden Ansprüche,
**dadurch gekennzeichnet,**
**dass** die Tülle (104) als eine flexible Ausstülpung der Beutelwand (102) ausgebildet ist.

5. Bergebeutel nach einem der vorangehenden Ansprüche,
**dadurch gekennzeichnet,**
**dass** die Tülle (104) an ihrem freien Ende eine Tüllenöffnung (105) trägt.

6. Bergebeutel (10) nach einem der vorangehenden Ansprüche,
**dadurch gekennzeichnet,**
**dass** die Beutelhauptöffnung (101) eine umlaufende Wandverstärkung trägt.

7. Bergebeutel (10) nach Anspruch 6,
**dadurch gekennzeichnet,**
**dass** die Wandverstärkung als ein federelastischer Offenhalter ausgebildet ist, der in einem die Beutelhauptöffnung (101) umlaufenden Führungskanal geführt und so vorgeformt ist, dass er im kraftunbeaufschlagten Zustand einen Ring bildet.

8. Bergebeutel (10) nach Anspruch 7,
**dadurch gekennzeichnet,**
**dass** der Ring ein offener Ring mit einem relativ zur Beutelwand (102) festen, ersten Ende und einem freien, zweiten Ende ist, sodass die lichte Weite der Beutelhauptöffnung (101) durch bei festgehaltenem ersten Ende auf das zweite Ende einwirkenden Zug verringerbar ist.

9. Laparoskopische Anordnung (14), umfassend
- einen Tubus (16) und
- einen im Lumen des Tubus (16) platzierten Bergebeutel (10) nach einem der vorangehenden Ansprüche
wobei der Bergebeutel (10) senkrecht zur Beutelhauptöffnung (101) zu einer Beutelrolle (12) derart aufgerollt ist,
dass das Entroll-Zugmittel (20) in der vom Beutelboden her aufgerollten Beutelrolle (12) parallel zur Beutelwand (102) verlaufend mit aufgerollt ist, sodass sein freies Ende im Bereich der Beutelhauptöffnung (101) aus der Beutelrolle (12) herausragt.

10. Laparoskopische Anordnung (14) nach Anspruch 9,
**dadurch gekennzeichnet,**
**dass** im Lumen des Tubus (16) der Beutelrolle (12) benachbart ein Schieber (18) angeordnet ist, der aus einem ersten Ende (161) des Tubus (16) herausragt, sodass die Beutelrolle (12) durch bei festgehaltenem Tubus (16) auf den Schieber (18) ausgeübten Schub am zweiten Ende (162) des Tubus (16) aus diesem herausschiebbar ist.

11. Laparoskopische Anordnung (14) nach Anspruch 10,
**dadurch gekennzeichnet,**
**dass** der Schieber (18) im Bereich der Beutelhauptöffnung (101) an der Beutelwand (102) fixiert ist.

## Claims

1. A retrieval bag (10) comprising a flexible bag wall (102) that is bounded at its upper end by a bag's main opening (101) has at least one spout (104) and forms with its lower third a bag's bottom (103) opposite the bag's main opening (101),
a flexible pull-to-unroll means (20) accessible from the outside of the bag being fixed to the bag's bottom, the pull-to-unroll means (20) being longer than the distance between its fixing point on the bag's bottom (103) and the bag's main opening (101),
**characterized in**
**that** the bag's bottom (103) comprises the spout (104) and the pull-to-unroll means (20) is fixed to the spout (104).

2. The retrieval bag (10) according to claim 1,
**characterized in**
**that** the pull-to-unroll means (20) is formed as a thread or a ribbon.

3. The retrieval bag (10) according to claim 2,
**characterized in**
**that** the pull-to-unroll means (20) is formed as a multi-strand thread or a multi-strand ribbon, the free ends of the strands being connected to one another or merge into one another to form at least one loop.

4. The retrieval bag retrieval bag (10) according to one of the preceding claims,
**characterized in**
**that** the spout (104) is formed as a flexible protrusion of the bag wall (102).

5. The retrieval bag (10) according to any of the preceding claims,
**characterized in**
**that** the spout (104) bears a spout opening (105) at its free end.

6. The retrieval bag (10) according to any of the preceding claims,
**characterized in**
**that** the bag's main opening (101) has a circumferential wall reinforcement.

7. The retrieval bag (10) according to claim 6,
**characterized in**
**that** the wall reinforcement is designed as a spring-elastic open-holder, which is guided in a guide channel surrounding the bag's main opening (101) and is preformed such that it forms a ring when no force is applied.

8. The retrieval bag (10) according to claim 7,
**characterized in**
**that** the ring is an open ring with a first end fixed relative to the bag wall (102) and a free second end, such that the clear width of the bag's main opening (101) can be reduced by applying tension to the second end while the first end is held in place.

9. Laparoscopic assembly (14) comprising
- a tube (16) and
- a retrieval bag (10) placed in the lumen of the tube (16) according to one of the preceding claims
wherein the retrieval bag (10) is rolled up perpendicular to the bag's main opening (101) to form a bag roll (12) such that the pull-to-enroll means (20) is wound up within the bag roll (12) - which is rolled up from the bag's bottom - running parallel to the bag wall (102), such that its free end protrudes from the bag roll (12) in the area of the bag's main opening (101).

10. The laparoscopic assembly (14) according to claim 9,
**characterized in**
**that** a pusher (18) is arranged in the lumen of the tube (16) adjacent to the bag roll (12), the pusher (18) protruding from a first end (161) of the tube (16), such that the bag roll (12) can be pushed out of the tube's (16) second end by applying thrust to the pusher while the tube (16) is held in place.

11. The laparoscopic assembly (14) according to claim 10,
**characterized in**
**that** the pusher (18) is fixed to the bag wall (102) in the region of the bag's main opening (101).

## Revendications

1. Un sac de récupération (10) comportant une paroi du sac (102) flexible qui est délimitée à son extrémité supérieure par une l'ouverture principale du sac (101), qui présente au moins un embout (104) et qui forme, par son tiers inférieur, un fond du sac (103), qui se trouve à l'opposé de l'ouverture principale du sac (101) et sur lequel est fixé un moyen de traction pour déroulement (20), que est flexible et accessible depuis l'extérieur du sac et qui est plus long que la distance entre son point de fixation sur le fond du sac (103) et l'ouverture principale du sac (101),
**caractérisé en ce**
**que** le fond du sac (103) comporte l'embout (104) et que le moyen de traction pour déroulement (20) est fixé à l'embout (104).

2. Le sac de récupération (10) selon la revendication 1,
**caractérisé en ce**
**que** le moyen de traction pour déroulement (20) est réalisé sous forme d'un fil ou d'un ruban.

3. Le sac de récupération (10) selon la revendication 2,
**caractérisé en ce**
**que** le moyen de traction pour déroulement (20) est réalisé sous forme d'un fil à plusieurs brins ou d'un ruban à plusieurs brins, dont les extrémités libres des brins sont reliées entre elles ou s'entrelace les unes dans les autres pour former au moins une boucle.

4. Le sac de récupération (10) selon l'une des revendications précédentes,
**caractérisé en ce**
**que** l'embout (104) est réalisé sous la forme d'un renflement flexible de la paroi du sac (102).

5. Le sac de récupération selon l'une des revendications précédentes,
**caractérisé en ce**
**que** l'embout (104) comporte une ouverture (105) à son extrémité libre.

6. Le sac de récupération (10) selon l'une des revendications précédentes,
**caractérisé en ce**
**que** l'ouverture principale du sac (101) comporte un renfort périphérique de paroi.

7. Le sac de récupération (10) selon la revendication 6,
**caractérisé en ce**
**que** le renfort de paroi est conçu comme un dispositif de maintien en position ouverte élastique, guidé dans un canal de guidage entourant l'ouverture principale du sac (101) et préformé de manière à former - à l'état non soumis à une force - un anneau.

8. Le sac de récupération (10) selon la revendication 7,
**caractérisé en ce**
**que** l'anneau est un anneau ouvert comportant une première extrémité fixe par rapport à la paroi du sac (102) et une deuxième extrémité libre, de sorte que la largeur intérieure de l'ouverture principale du sac (101) peut être réduite par une traction exercée sur la deuxième extrémité tandis que la première extrémité est maintenue fixe.

9. Un dispositif laparoscopique (14) comprenant
- un tube (16) et
- un sac de récupération (10) selon l'une des revendications précédentes placé dans le lumen du tube (16),
le sac de récupération (10) étant enroulé perpendiculairement à l'ouverture principale du sac (101) pour former un rouleau du sac (12) de telle sorte que le moyen de traction pour déroulement (20) est enroulé dans le rouleau du sac (12) - enroulé à partir du fond du sac - parallèlement à la paroi du sac (102), de sorte que son extrémité libre dépasse du rouleau de sac (12) au niveau de l'ouverture principale du sac (101).

10. Le dispositif laparoscopique (14) selon la revendication 9,
**caractérisé en ce**
**qu'**un poussoir (18) est disposé dans le lumen du tube (16) à proximité du rouleau du sac (12), lequel poussoir (18) dépasse d'une première extrémité (161) du tube (16), de sorte que le rouleau du sac (12) peut être poussé hors du tube (16) au niveau de la deuxième extrémité (162) du tube (16) par une poussée exercée sur le coulisseau (18).

11. Le dispositif laparoscopique (14) selon la revendication 10,
**caractérisé en ce**
**que** le poussoir (18) est fixé à la paroi du sac (102) au niveau de l'ouverture principale du sac (101).
